(19) Europäisches Patentamt
European Patent Office
Office européen des brevets

(11) Numéro de publication: **0 354 845 A1**

# (12) DEMANDE DE BREVET EUROPEEN

(21) Numéro de dépôt: **89402247.4**

(22) Date de dépôt: **09.08.89**

(51) Int. Cl.[5]: **C 07 F 15/00**
B 01 J 31/24, C 07 C 45/50

(30) Priorité: **11.08.88 FR 8810820**

(43) Date de publication de la demande:
**14.02.90 Bulletin 90/07**

(84) Etats contractants désignés:
**AT BE CH DE ES FR GB GR IT LI LU NL SE**

(71) Demandeur: **NORSOLOR**
**Tour Aurore Place des Reflets**
**F-92080 Paris la Défense 2- Cedex 5 (FR)**

(72) Inventeur: **Paumard, Eric**
**338, rue de l'abbé Touba**
**F-57450 Cappel (FR)**

**Mortreux, André**
**17, rue Gambetta**
**F-59510 Hem (FR)**

**Petit, Francis**
**27 Allée de la Clairière**
**F-59650 Villeneuve d'Ascq (FR)**

(74) Mandataire: **Dubost, Thierry**
**c/o NORSOLOR Service Propriété Industrielle B.P. 57**
**F-62670 Mazingarbe (FR)**

## (54) Hydrures de platine de structure bimétallique pontée, leur procédé de préparation et leur application à la catalyse de réactions chimiques.

(57) Hydrures de platine de struture bimétallique pontée. Ils sont représentés par la formule générale :

(I) 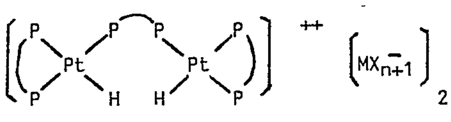

dans laquelle =
- M est un métal à l'état de valence n au moins égal à 2,
- X est un atome d'halogène, et
- P P constitue une représentation schématique d'un ligand de formule générale :

(II) 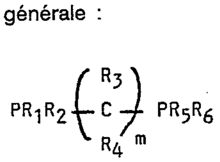

dans laquelle =
. $R_1$, $R_2$, $R_5$ et $R_6$, identiques ou différents, sont choisis parmi les radicaux hydrocarbonés aliphatiques ou cycloaliphatiques possédant de 1 à 8 atomes de carbone et les radicaux hydrocarbonés aromatiques possédant de 6 à 10 atomes de carbone,
. $R_3$ et $R_4$, identiques ou différents, sont choisis parmi l'atome d'hydrogène et les radicaux hydrocarbonés aliphatiques, possédant de 1 à 8 atomes de carbone, le cas échéant fonctionnalisés et/ou formant ensemble un cycle et,
. m est un nombre entier supérieur ou égal à 4.

Application d'un hydrure de platine comme composant de système catalytique destiné à catalyser une réaction chimique.

EP 0 354 845 A1

## Description

### HYDRURES DE PLATINE DE STRUCTURE BIMETALLIQUE PONTEE, LEUR PROCEDE DE PREPARATION ET LEUR APPLICATION A LA CATALYSE DE REACTIONS CHIMIQUES.

La présente invention se rapporte à de nouveaux hydrures de platine, à leur procédé de préparation et à leur utilisation comme composants de systèmes catalytiques destinés à catalyser des réactions chimiques, notamment des réactions d'hydroformylation de composés éthyléniquement insaturés.

Un premier objet de la présente invention consiste en une nouvelle classe d'hydrures de platine de struture bimétallique pontée, représentés par la formule générale :

(I) 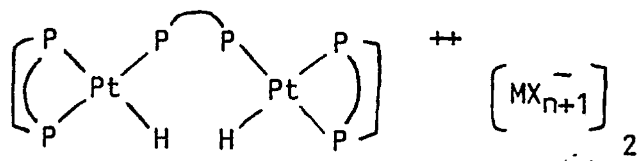

dans laquelle =
- M est un métal à l'état de valence n au moins égal à 2,
- X est un atome d'halogène, et
- P P constitue une représentation schématique d'un ligand de formule générale :

(II) 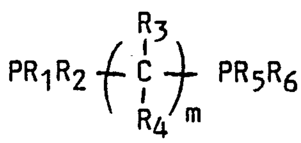

dans laquelle =
. $R_1$, $R_2$, $R_5$ et $R_6$, identiques ou différents, sont choisis parmi les radicaux hydrocarbonés aliphatiques ou cycloaliphatiques possédant de 1 à 8 atomes de carbone et les radicaux hydrocarbonés aromatiques possédant de 6 à 10 atomes de carbone,
. $R_3$ et $R_4$, identiques ou différents, sont choisis parmi l'atome d'hydrogène et les radicaux hydrocarbonés aliphatiques, possédant de 1 à 8 atomes de carbone, le cas échéant fonctionnalisés et/ou formant ensemble un cycle et,
. m est un nombre entier supérieur ou égal à 4.

Dans la formule (I) des hydrures de platine selon l'invention, X désigne de préférence le chlore, n est de préférence égal à 2 ou à 3, et le métal M est de préférence choisi parmi les groupes IVB, VIII, IB, IIB, IIIA et IVA de la Classification Périodique. De manière plus particulièrement préférée, M peut être choisi parmi le fer (II), le zinc, l'étain, le cuivre, l'aluminium et le titane (III).

Dans la formule (II) du ligand, il est préférable que $R_1 = R_5$ et que $R_2 = R_6$, et plus particulièrement que $R_1 = R_2 = R_5 = R_6$. A titre d'exemples on peut citer les radicaux méthyle, éthyle, isopropyle, tertiobutyle, néopentyle, cyclohexyle, phényle, etc. Lorsque les radicaux $R_3$ et $R_4$ sont fonctionnalisés, ils peuvent être porteurs notamment d'une fonction telle que thiol, alcool, thioether, amine, imine, acide, ester, amide ou éther.

Une classe de ligands plus particulièrement préférée est celle représentée par la formule générale :

(III) $PR_2 - CH_2 -(CHR')_{m-2}\ CH_2-PR_2$ dans laquelle =
- R est choisi parmi les radicaux hydrocarbonés aliphatiques ou cycloaliphatiques possédant de 1 à 8 atomes de carbone et les radicaux hydrocarbonés aromatiques possédant de 6 à 10 atomes de carbone.
- R' est choisi parmi les radicaux hydrocarbonés aliphatiques possédant de 1 à 8 atomes de carbone, le cas échéant fonctionnalisés (la fonction portée étant choisie telle que ci-dessus), et
- m est un nombre entier supérieur ou égal à 4.

Des exemples de tels ligands sont notamment le (1S,2S)(+)trans-1,2-bis(diphénylphosphinométhyl)-cyclohexane, le 1,4-bis(diphénylphosphino)-butane et l'isopropylidène-dihydroxy-2,3-bis-(diphénylphosphino)1,4-butane.

Les hydrures de platine selon la présente invention peuvent être préparés selon divers procédés. Un premier procédé comprend :
- dans une première étape, la réduction d'un solvant comprenant au moins un carbonate d'alcène dans une cellule électrochimique dont l'anode est en métal M de manière à former une combinaison chimique entre le métal M et le carbonate d'alcène puis
- la réaction de cette combinaison avec au moins un complexe de platine de formule

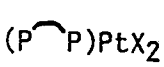

dans laquelle X et

P⌒P

ont les significations explicitées précèdemment, et enfin,
- dans une dernière étape l'application d'une atmosphère d'hydrogène.

Ainsi ce procédé comprend, en première étape, la formation d'une combinaison chimique entre le métal M et un carbonate d'alcène pouvant être choisi notamment parmi ceux de propylène, d'éthylène, de 1,2-butylène et de 1,2-hexylène. Selon un mode de réalisation particulier de ce procédé, la réduction du solvant peut être effectuée en présence d'une faible quantité d'un sel conducteur soluble dans ledit solvant tel que, par exemple, l'hexafluorophosphate de tétra-n-butylammonium. La présence de ce sel conducteur permet avantageusement d'accélérer la réduction du solvant, en particulier à température modérée. La phase de réduction électrochimique selon l'invention est généralement effectuée à une température comprise entre 10°C et 70°C environ et en maintenant la cellule électrochimique sous atmosphère d'un gaz inerte tel que l'azote, l'argon ou le monoxyde de carbone ; il est préférable du point de vue des utilisations ultérieures des hydrures de platine ainsi préparés, notamment en tant que composants de systèmes catalytiques pour des réactions chimiques, que cette réduction électrochimique soit effectuée en l'absence d'hydrogène. Le solvant électrochimique utilisé dans cette étape du procédé selon l'invention comprend nécessairement au moins un carbonate d'alcène tel que défini précédemment. Il peut comprendre en outre, en mélange avec ce dernier, un autre solvant tel que par exemple un hydrocarbure aromatique (benzène, toluène, xylène, etc.). Pour la mise en oeuvre du procédé selon l'invention, on préférera utiliser un solvant comprenant au moins 10% environ en volume de carbonate d'alcène.

Dans le cadre de ce premier procédé, deux variantes peuvent être envisagées pour la réduction électrochimique. Selon la première variante la cellule électrochimique comprend, outre l'anode en métal M, une cathode et une électrode de référence et la réduction est effectuée en portant la cathode à un potentiel inférieur ou égal à -1,5 volt environ par rapport à l'électrode de référence et en imposant ce potentiel pendant une durée suffisante pour assurer la production de la quantité voulue de la combinaison chimique "M-carbonate d'alcène" ; dans ce cas l'électrode de référence peut être, par exemple, l'une des électrodes $Ag/AgCl/Cl^-$, $Ag/Ag^+$ et $Hg/Hg_2Cl_2$ (calomel). Selon la seconde variante, la cellule électrochimique comporte l'anode en métal M et une cathode entre lesquelles on applique une différence de potentiel supérieure ou égale à 10 volts environ, et à maintenir cette différence de potentiel pendant une durée suffisante pour assurer la production de la quantité voulue de la combinaison "M-carbonate d'alcène". Comme dans la variante précédente, l'anode en métal M passe progressivement en solution.

Comme exemples de cathodes utilisables dans ce premier procédé on peut citer d'une part les cathodes en graphite et d'autre part des cathodes en métal inaltérable vis-à-vis du solvant, tel que platine ou acier inoxydable.

Ce premier procédé selon l'invention comprend en outre la réaction de la combinaison chimique formée entre le métal M et le carbonate d'alcène, dite par abréviation "M-carbonate d'alcène", avec le complexe de platine de formule

(P⌒P)$PtX_2$.

Cette réaction est de préférence effectuée dans au moins un solvant dudit complexe, à une température comprise entre 10°C et la température d'ébullition dudit solvant. Parmi les solvants du complexe de platine on peut citer notamment les hydrocarbures aromatiques (tels que par exemple le benzène, le toluène et les xylènes) et les carbonates d'alcène, notamment ceux dont le groupe alcène possède de 2 à 6 atomes de carbone. La durée de la réaction entre la combinaison "M-carbonate d'alcène" et le complexe de platine peut varier, selon les règles usuelles bien connues de l'homme de l'art, en fonction de la température de réaction choisie et de la concentration des espèces réactives dans le solvant. A titre d'exemple cette durée n'est généralement pas supérieure à 20 minutes lorsque la température de réaction est égale à 80°C.

Plusieurs modes de réalisation de cette réaction peuvent être envisagés dans le cadre de ce permier procédé selon l'invention. Un premier mode de réalisation consiste à former une combinaison "M-carbonate d'alcène", par réduction électrochimique, puis à introduire le complexe de platine dans le milieu dans lequel ladite combinaison a été formée, ledit milieu comprenant déjà le solvant nécessaire à la réaction. Un second mode de réalisation consiste à former une combinaison "M-carbonate d'alcène", par réduction électrochimique, à isoler (sous forme de poudre) ladite combinaison du milieu dans lequel elle a été formée (par exemple en filtrant, lavant et sèchant le précipité qui se forme dans la cellule électrochimique), puis à introduire ladite poudre dans une solution du complexe de platine dans le solvant nécessaire à la réaction. Enfin un troisième mode de réalisation consiste à former la combinaison "M-carbonate d'alcène" en présence du solvant et du complexe de platine, par exemple réduction électrochimique ; dans ce cas on opère pendant une durée suffisante pour assurer la production d'une quantité d'électricité au moins égale à 0,2 Faraday par atome-gramme de platine. Quel que soit le mode de réalisation choisi pour le procédé selon l'invention, il est souhaitable que la concentration du complexe de platine dans le solvant de la réaction soit comprise entre

0,001 et 0,2 mole par litre environ.

Un second procédé de préparation des hydrures de platine selon l'invention comprend la réaction d'un composé de métal M avec un complexe de platine de formule

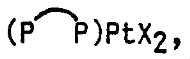

dans laquelle X et

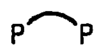

ont les significations explicitées précédemment, en présence d'un solvant comprenant au moins un carbonate d'alcène, suivie de l'application d'une atmosphère d'hydrogène. Le composé de métal M soumis à cette réaction est de préférence un composé comprenant au moins une liaison covalente M-O, tel qu'un oxyde, un alcoolate ou un éther métallique.

Ce second procédé comprend donc, dans un premier temps, une réaction chimique dans un milieu solvant comprenant un carbonate d'alcène, dont le groupe alcène possède de préférence de 2 à 6 atomes de carbone, le cas échéant en mélange avec un hydrocarbure aromatique tel que benzène, toluène ou xylène ; de préfèrence, comme dans le premier procédé selon l'invention, ledit milieu solvant comprend au moins 10% en volume de carbonage d'alcène. Les autres conditions réactionnelles, telles que température et durée, seront choisies de même.

Dans le premier comme dans le second procédé selon l'invention, les quantités de la combinaison "M-carbonate d'alcène" et du complexe de platine mises en jeu au cours de la réaction sont choisies de telle sorte que le rapport atomique M/Pt soit compris entre 0,5 et 2 environ, et de préfèrence égal à 1. Dans le premier comme dans le second procédé selon l'invention, la réaction entre la combinaison "M-carbonate d'alcène" et le complexe de platine est suivie d'une étape d'hydrogénation, c'est-à-dire d'application d'une atmosphère d'hydrogène ; cette dernière étape du procédé a généralement lieu sous pression (pouvant atteindre 200 bars environ) et le cas échéant sous température élevée (pouvant atteindre 150°C).

Les hydrures de platine selon l'invention possèdent une structure bimétallique pontée de formule (I) authentifiée par les techniques analytiques suivantes :

- résonnance magnétique nucléaire du phosphore 31, du proton, du platine 195 et du métal M.
- spectroscopie infrarouge établissant la présence d'une fine bande d'absorption de la liaison platine-hydrogène à 2010 $cm^{-1}$.
- spectrométrie de masse.

Ces hydrures de platine de struture bimétallique pontée possèdent une utilité remarquable en tant que composants de systèmes catalytiques pour des réactions chimiques, notamment pour des réactions d'hydroformylation de composés éthylèniquement insaturés. Ainsi un autre objet de la présente invention consiste en l'application ce ces hydrures à la fabrication d'aldéhydes par hydroformylation d'un composé organique à insaturation éthylénique, caractérisé en ce que l'on fait réagir, à une température comprise entre 10°C et 300°C environ et sous une pression comprise entre 10 et 350 bars environ, ledit composé organique avec un mélange de monoxyde de carbone et d'hydrogène en présence d'une quantité efficace dudit hydrure. Une quantité efficace de système catalytique est généralement telle que le rapport molaire du composé organique à insaturation éthylénique au platine soit compris entre 100 et 10.000. Le rapport molaire $CO/H_2$ dans le mélange de monoxyde de carbone et d'hydrogène intervenant dans la réaction d'hydroformylation selon l'invention est généralement compris entre 0,5 et 2 environ.

Parmi les composés organiques à insaturation éthylénique pouvant être soumis à la réaction d'hydroformylation selon l'invention, on peut citer notamment :

- les oléfines ayant de 2 à 12 atomes de carbone, telles que notamment le propylène, le butène-1, l'hexène-1, le méthyl-4-pentène-1, l'octène-1, etc.
- les composés vinylaromatiques tels que le styrène, l'alphaméthylstyrène.
- les diènes tels que par exemple le vinyl-4-cyclohexène.

La durée de la réaction d'hydroformylation selon l'invention est généralement comprise entre 0,5 et 30 heures environ, selon la pression et la température choisies.

Le procédé d'hydroformylation selon l'invention présente l'intérêt de permettre l'obtention, avec un excellent taux de transformation, d'un mélange d'aldéhydes normaux et ramifiés dans lequel la proportion d'alhydes normaux est particulièrement élevée.

Les exemples ci-après sont donnés à titre illustratif et non limitatif de la présente invention :

EXEMPLE 1

Dans une cellule électrochimique en verre, on introduit sous azote 64 mg de complexe $LPtCl_2$, le ligand L étant l'isopropylidène-dihydroxy-2,3-bis(diphénylphosphino)-1,4-butane, puis le solvant constitué d'un mélange de 15 $cm^3$ de benzène et de 10 $cm^3$ de carbonate de propylène. Après dissolution complète du

complexe, on plonge dans le solvant la cathode (constituée d' un un panier de platine) et l'anode (constituée d'un cylindre d'étain), puis l'électrode de référence (Ag/AgCl/N($C_4H_9$)$_4$Cl 0,1M dans le carbonate de propylène). La température étant égale à 20° C, on fixe le potentiel de réduction à -1,85 volt entre la cathode et l'électrode de référence et l'électroréduction est arrêtée quant la quantité de courant ayant traversé le circuit correspond à un rapport atomique Sn/Pt est égal à 1. Au cours de la coulomètrie, la solution passe de l'incolore au marron en passant par le jaune. La solution est alors transférée dans un réaction autoclave de 50 cm$^3$ préalablement dégazé. On charge alors le réacteur en hydrogène jusqu'à une pression de 100 bars, puis le réacteur est chauffé à une température atteignant jusqu'à 100° C et l'agitation est mise en marche. Après environ 2 heures, l'autoclave est refroidi et le mélange gazeux éliminé. La solution est transféré dans un tube de Schlenk et stockée sous argon.

Le composé préparé a fait l'objet des analyses spectroscopiques suivantes :

- la résonance magnétique nucléaire du phosphore 31 à 162 MHz par préférence à l'acide phosphorique permet d'observer des déplacement chimiques à 4,22 ppm, 8,15 ppm et 13,8 ppm.
- la résonance magnétique nucléaire du proton à 400 MHz par référence au tétraméthylsulfure permet d'observer un déplacement chimique à -5,15 ppm.
- la résonance magnétique nucléaire du platine 195 à 86 MHz par référence à à $H_2PtCl_6$ permet d'observer un déplacement chimique à -5390 ppm.
- la réson ance magnétique nucléaire de l'étain 119 à 149 MHz par référence au tétraméthylétain fait apparaître un déplacement chimique à -36 ppm.
- la spectrométrie de masse permet d'établir que le composé préparé, de masse globale égale à 2337 grammes, répond à la formule générale :

$C_{93}H_{98}Cl_6O_6P_6Pt_2Sn_2$

L'ensemble des analyses exposées ci-dessus permet donc d'établir la formule détaillée :

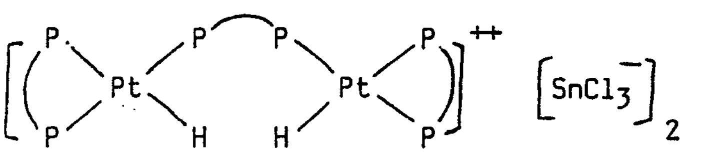

dans laquelle P P désigne l'isopropylidène-dihydroxy-2,3-bis(diphénylphosphino)-1,4-butane. En accord avec la nomenclature internationale ce composé est dénommé : dihydrido[μ-(2,2-diméthyl-1,3-dioxolane-4,5diyl)bis(méthylène) bis [diphenyl phosphine] -P : P']] bis [[ (2,2-diméthyl-1,3-dioxolane-4,5-diyl)bis(méthylène) bis[diphenyl phosphine] -P : P']] diplatinum (II) bis trichlorostannate.
et peut encore être schématisé comme suit :

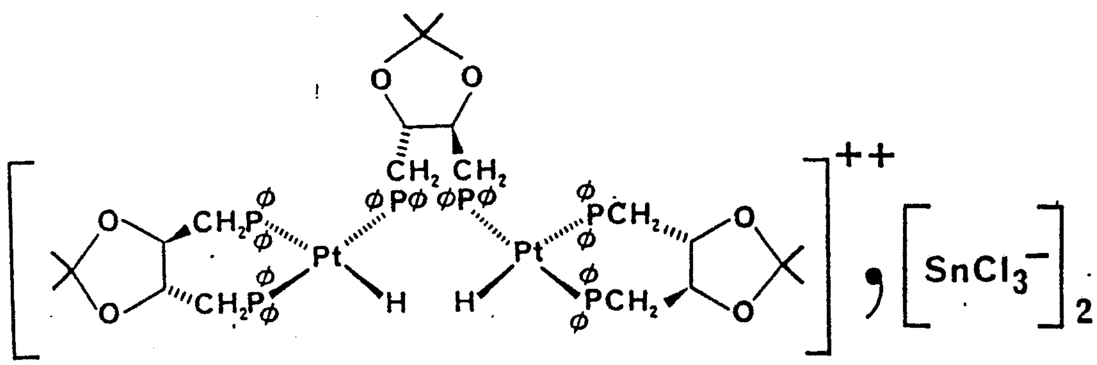

EXEMPLE 2

La solution d'hydrure de platine préparée conformément à l'exemple 1 est introduite dans un réacteur autoclave de 100 cm$^3$ en acier inoxydable équipé d'un système d'agitation par barreau aimanté, puis on ajoute du styrène en quantité telle que le rapport molaire styrène/Pt soit égal à 100. Le réacteur est chauffé jusqu'à la température de 80° C puis on charge le gaz de synthèse constitué d'un mélange équimolaire de monoxyde de carbone et d'hydrogène. Enfin le mélange est agité et la réaction est poursuivie à 80° C sous une pression de 50 bars pendant 24 heures. On obtient alors, avec un taux de transformation TT (exprimé en pourcent), un mélange comprenant de l'éthylbenzène, du phényl-2 propanal et du phényl-3 propanal. L'analyse de ce mélange permet de déterminer d'une part la proportion en poids d'éthylbenzène EB (exprimée en pourcent) et d'autre par le rapport molaire n/b de l'aldéhyde normal à l'aldéhyde branché. Le tableau I ci-après rassemble les résultats obtenus.

EXEMPLE 3

On répète le processus opératoire de l'exemple 2, aux exceptions suivantes près : la réaction est effectuée à 90°C sous une pression de 100 bars pendant 8 heures. Les résultats obtenus sont indiqués au tableau I ci-après.

EXEMPLE 4

On répète le procédé de préparation de l'exemple 1 à l'exception du fait que le solvant est constitué d'un mélange de 10 $cm^3$ de benzène et 15 $cm^3$ de carbonate de propylène. La solution d'hydrure de platine ainsi obtenu est alors utilisée pour l'hydroformylation du styrène dans les conditions opératoires de l'exemple 2. Les résultats obtenus sont indiqués au tableau I ci-après.

EXEMPLES 5 à 9

On répète le procédé de préparation de l'exemple 1 à l'exception de la nature de l'anode, pour laquelle l'étain est rempacé par un autre métal indiqué au tableau I. Les analyses spectroscopiques effectuées comme à l'exemple 1 indiquent que le produit formé est bien un hydrure de platine de structure bimétallique pontée de formule (I). L'hydroformylation du styrène est alors effectuée conformément au processus opératoire de l'exemple 3 à l'exception de la durée de la réaction, qui est portée de 8 à 19 heures. Les résultats obtenus sont indiqués au tableau I ci-après.

TABLEAU I

| exemple | anode | TT | EB | n/b |
|---|---|---|---|---|
| 2 | Sn | 67 | 6 | 4,3 |
| 3 | Sn | 100 | 8 | 4,0 |
| 4 | Sn | 53 | 4 | 5,5 |
| 5 | Al | 90 | 17 | 1,9 |
| 6 | Ti | 80 | 14,5 | 2,5 |
| 7 | Fe | 100 | 3 | 5,0 |
| 8 | Cu | 45 | 18 | 4,8 |
| 9 | Zn | 100 | 4 | 5,3 |
| 10 | Fe | 100 | 3 | 9,3 |
| 11 | Fe | 100 | 15,5 | 8,1 |

EXEMPLE 10

On répète le procédé de préparation d'hydrure de platine de l'exemple 7 (anode en fer) à l'exception du fait que le solvant est consti tué de 25 $cm^3$ d'un mélange à 75% en volume de benzène. L'hydroformylation du styrène est alors effectuée conformément au processus opératoire de l'exemple 3 à l'exception de la durée de la réaction, qui est portée de 8 à 24 heures. Les résultats obtenus sont indiqués au tableau I ci-avant.

EXEMPLE 11

On répète l'expérience de l'exemple 10 à l'exception de la réaction d'hydroformylation, qui est effectuée à 110°C pendant 21 heures. Les résultats obtenus sont indiqués au tableau I ci-avant.

EXEMPLE 12 à 16

On fait réagir 64 mg de complexe $LPtCl_2$, le ligand étant l'isopropylène-dihydroxy-2,3-bis (diphénylphosphino)-1,4-butane, dans 25 $cm^3$ d'un mélange solvant comprenant 75% en volume de benzène et 25% en volume de carbonate de propylène, avec un composé de fer (de formule indiquée au tableau II ci-après) en quantité telle que le rapport atomique Fe/Pt soit égal à 1. Le mélange réactionnel est ensuite mis en présence, dans un réacteur autoclave d'hydrogène sous une pression de 100 bars, à une température de 100°C pendant 2 heures. A l'issue de ce temps, l'autoclave est refroidi et le mélange gazeux éliminé. La solution est transférée dans un tube de Schlenk et stockée sous argon. Les analyses spectroscopiques effectuées comme à l'exemple 1 indiquent que le produit formé est bien un hydrure de platine de structure bimétallique pontée de formule (I), l'anion associé étant $FeCl_3$.

Chaque solution d'hydrure de platine est alors utilisée pour l'hydroformylation du styrène selon le processus opératoire de l'exemple 2, aux exceptions suivantes près : la réaction est effectuée à 90°C sous une pression de 100 bars pendant 24 heures (18 heures seulement pour l'exemple 13). Les résultats obtenus sont indiqués au tableau II ci-après.

TABLEAU II

| exemple | composé de fer | TT | EB | n/b |
|---|---|---|---|---|
| 12 | $Fe_2O_3$ | 100 | 5,7 | 11,9 |
| 13 | $Fe_3O_4$ | 100 | 4,8 | 13,2 |
| 14 | $Fe(OH)(OCOCH_3)_2$ | 92 | 2,5 | 6,0 |
| 15 | $Fe(OCOCH_3)_2$ | 97 | 2,5 | 9,8 |
| 16 | $Fe(OCH_3)_2$ | 100 | 4,0 | 5,6 |

**Revendications**

1. Hydrures de platine de struture bimétallique pontée, représentés par la formule générale :

(I) 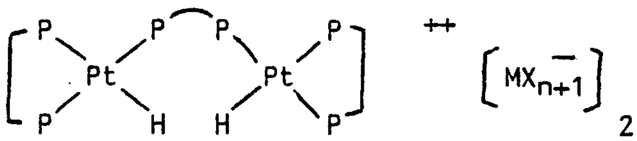

dans laquelle = - M est un métal à l'état de valence n au moins égal à 2,
- X est un atome d'halogène, et
- P P constitue une représentation schématique d'un ligand de formule générale :

(II) 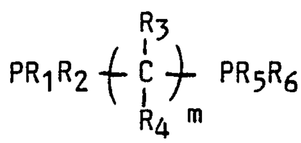

dans laquelle =
. $R_1$, $R_2$, $R_5$ et $R_6$, identiques ou différents, sont choisis parmi les radicaux hydrocarbonés aliphatiques ou cycloaliphatiques possédant de 1 à 8 atomes de carbone et les radicaux hydrocarbonés aromatiques possédant de 6 à 10 atomes de carbone,
. $R_3$ et $R_4$, identiques ou différents, sont choisis parmi l'atome d'hydrogène et les radicaux hydrocarbonés aliphatiques, possédant de 1 à 8 atomes de carbone, le cas échéant fonctionnalisés et/ou formant ensemble un cycle et,
. m est un nombre entier supérieur ou égal à 4.

2. Hydrures de platine selon la revendication 1, caractérisés en ce que X est le chlore.

3. Hydrures de platine selon l'une des revendications 1 à 2, caractérisés en ce que le métal M est choisi parmi les groupes IVB, VIII, IB, IIB, IIIA et IVA de la Classification Périodique.

4. Hydrures de platine selon l'une des revendications 1 à 3, caractérisés en ce que le métal M est choisi parmi le fer (II), le zinc, l'étain, le cuivre, l'aluminium et le titane (III).

5. Hydrures de platine selon l'une des revendications 1 à 4, caractérisés en ce que le ligand

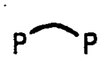

est l'isopropylène-dihydroxy-2,3-bis(diphénylphosphino)-1,4-butane.

6. Procédé de préparation d'hydrures de platine selon la revendication 1, caractérisé en ce qu'il comprend :
- dans une première étape, la réduction d'un solvant comprenant au moins un carbonate d'alcène dans une cellule électrochimique dont l'anode est en métal M de manière à former une combinaison chimique entre le métal M et le carbonate d'alcène, puis
- la réaction de cette combinaison avec au moins un complexe de platine de formule

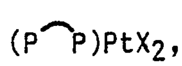

et enfin - dans une dernière étape l'application d'une atmosphère d'hydrogène.

7. Procédé de préparation selon la revendication 6, caractérisé en ce que le groupe alcène du

carbonate d'alcène comprend de 2 à 6 atomes de carbone.

8. Procédé selon l'une des revendications 6 et 7, caractérisé en ce que la réduction du solvant est effectuée en présence d'une faible quantité d'un sel conducteur soluble dans le solvant.

9. Procédé selon l'une des revendications 6 à 8, caractérisé en ce que la réduction du solvant est effectuée à une température comprise entre 10°C et 70°C et en maintenant la cellule électrochimique sous atmosphère d'un gaz inerte.

10. Procédé selon l'une des revendications 6 à 9, caractérisé en ce que le solvant comprend en outre un hydrocarbure aromatique en mélange avec le carbonate d'alcène.

11. Procédé selon la revendication 10, caractérisé en ce que ledit solvant comprend au moins 10% en volume de carbonate d'alcène.

12. Procédé selon l'une des revendications 6 à 11, caractérisés en ce que la cellule électrochimique comprend, outre l'anode en métal M, une cathode et une électrode de référence, en ce que la réduction est effectuée en portant la cathode à un potentiel inférieur ou égal à - 1,5 volt par rapport à l'électrode de référence, et en imposant ce potentiel pendant une durée suffisante pour assurer la production de la quantité voulue de la combinaison chimique "M-carbonate d'alcène".

13. Procédé de préparation selon l'une des revendications 6 à 11, caractérisés en ce que la cellule électrochimique comporte l'anode en métal M et une cathode entre lesquelles on applique une différence de potentiel supérieure ou égale à 10 volts pendant une durée suffisante pour assurer la production de la quantité voulue de ladite combinaison chimique "M-carbonate d'alcène".

14. Procédé de préparation selon l'une des revendications 6 à 13, caractérisés en ce que la réaction de la combinaison chimique "M-carbonate d'alcène" et du complexe de platine est effectuée dans un solvant, la concentration du complexe de platine dans ledit solvant étant compris entre 0,001 et 0,2 mole par litre.

15. Procédé de préparation d'hydrures de platine selon la revendication 1, caractérisé en ce qu'il comprend la réaction d'un composé de métal M avec un complexe de platine de formule

$$(P\frown P)PtX_2,$$

en présence d'un solvant comprenant au moins un carbonate d'alcène, suivie de l'application d'une atmosphère d'hydrogène.

16. Procédé de préparation selon la revendication 15, caractérisé en ce que le composé de métal M comprend au moins une liaison covalente M-O.

17. Application d'un hydrure de platine selon la revendication 1 comme composant de système catalytique destiné à catalyser une réaction chimique.

18. Application selon la revendication 17 à la fabrication d'aldéhydes par hydroformylation d'un composé organique à insaturation éthylénique.

Office européen des brevets

# RAPPORT DE RECHERCHE EUROPEENNE

Numero de la demande

EP 89 40 2247

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | US-A-4 522 932 (H.L. MITCHELL, III)<br>* Colonne 12 * | 1 | C 07 F 15/00<br>B 01 J 31/24<br>C 07 C 45/50 |
| A | JOURNAL OF ORGANOMETALLIC CHEMISTRY, vol. 236, 1982, pages C33-C36, Elsevier Sequoia S.A., Lausanne, CH; M.P. BROWN et al.: "Preparation and X-ray crystal structure of bis[bis(di-phenylphosphino)methanido]platinum(II), [Pt(Ph2PCHPPh2)2]"<br>* En entier * | 1 | |
| A | US-A-3 519 663 (S. O'BRIEN)<br>* Revendications 1-12 * | 1 | |

DOMAINES TECHNIQUES RECHERCHES (Int. Cl.4)

C 07 F 15/00

Le présent rapport a été établi pour toutes les revendications

| Lieu de la recherche | Date d'achèvement de la recherche | Examinateur |
|---|---|---|
| LA HAYE | 12-10-1989 | PAUWELS G.R.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)